(19) Europäisches Patentamt
European Patent Office
Office européen des brevets

(11) Veröffentlichungsnummer: **0 400 427 B1**

(12) **EUROPÄISCHE PATENTSCHRIFT**

(45) Veröffentlichungstag der Patentschrift: **05.04.95**

(51) Int. Cl.[6]: **C07D 209/48**, A01N 37/32, A01N 43/38

(21) Anmeldenummer: **90109522.4**

(22) Anmeldetag: **19.05.90**

Die Akte enthält technische Angaben, die nach dem Eingang der Anmeldung eingereicht wurden und die nicht in dieser Patentschrift enthalten sind.

# (54) Herbizide 5-(N-3,4,5,6-tetrahydrophthalimido)-zimtsäureester, Verfahren zur Herstellung und ihre Verwendung.

(30) Priorität: **31.05.89 DE 3917676**
**22.09.89 DE 3931615**

(43) Veröffentlichungstag der Anmeldung:
**05.12.90 Patentblatt 90/49**

(45) Bekanntmachung des Hinweises auf die Patenterteilung:
**05.04.95 Patentblatt 95/14**

(84) Benannte Vertragsstaaten:
**AT BE CH DE DK ES FR GB IT LI NL SE**

(56) Entgegenhaltungen:
**DE-A- 3 724 399**

(73) Patentinhaber: **BASF Aktiengesellschaft**
**Carl-Bosch-Strasse 38**
**D-67063 Ludwigshafen (DE)**

(72) Erfinder: **Rueb, Lothar, Dr.**
**Am Schoeneck 11**
**D-6720 Speyer (DE)**
Erfinder: **Eicken, Karl, Dr.**
**Am Huettenwingert 12**
**D-6706 Wachenheim (DE)**
Erfinder: **Westphalen, Karl-Otto, Dr.**
**Mausbergweg 58**
**D-6720 Speyer (DE)**
Erfinder: **Wuerzer, Bruno, Dr.**
**Ruedigerstrasse 13**
**D-6701 Otterstadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue 5-(N-3,4,5,6-tetrahydrophthalimido)-zimtsäureester der allgemeinen Formel I

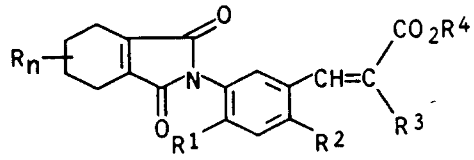

I

in der die Substituenten und der Index folgende Bedeutung haben:

- $R^1$ Wasserstoff oder Fluor,
- $R^2$ Halogen,
- $R^3$ Wasserstoff, Halogen oder eine $C_1$-$C_4$-Alkylgruppe,
- $R^4$ Wasserstoff, eine $C_1$-$C_6$-Alkylgruppe, welche durch ein oder zwei $C_1$-$C_4$-Alkoxy- oder $C_1$-$C_4$-Alkylthiogruppen substituiert sein kann, eine $C_3$-$C_7$-Cycloalkylgruppe, eine $C_3$-$C_6$-Alkenylgruppe, eine $C_3$-$C_6$-Alkinylgruppe oder ein Benzylrest,
- R $C_1$-$C_4$-Alkyl,
- n 1 oder 2,

wobei die Formel I sämtliche isomeren Formen dieser Verbindungen umfaßt.

Des weiteren betrifft die Erfindung Verfahren zur Herstellung dieser Verbindungen sowie deren Verwendung als herbizide Mittel und zur Entblätterung (Desikkation) von Baumwolle.

Die DE-A 37 24 399 beschreibt herbizid wirksame Phenylalkenylcarbonsäuren sowie Alkyl-, Cycloalkyl-, Alkoxyalkyl- und Alkylthioalkyl-Ester dieser Verbindungen, die sich von den 5-(N-3,4,5,6-tetrahydrophthalimido)-zimtsäureestern hauptsächlich durch die fehlende Alkylgruppe R unterscheiden.

Des weiteren sind aus der japanischen Offenlegungsschrift JP-A Kokai 27962/1986 Ester und Amide der Struktur I' bekannt,

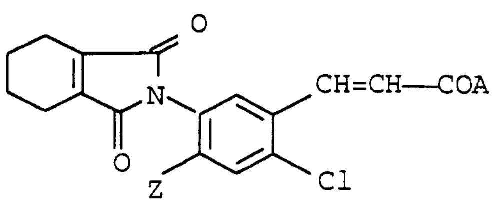

I'

welche jedoch hinsichtlich der benötigten Aufwandmengen zu wünschen übrig lassen.

Der Erfindung lag die Aufgabe zugrunde, besonders geeignete herbizide Verbindungen bereitzustellen.

Es wurde nun gefunden, daß die eingangs definierten 5-(N-3,4,5,6-Tetrahydrophthalimido)-zimtsäureester der Formel I auch bei geringen Aufwandmengen eine gute herbizide Wirksamkeit besitzen.

Man erhält die Verbindungen I beispielsweise dadurch, daß man ein Zimtsäurederivat II in einem aprotisch polaren organischen Lösungsmittel in an sich bekannter Weise (Houben Weyl Bd. X/2, 747) in Anwesenheit einer Base mit einem Alkohol der Formel III umsetzt, den so erhaltenen Nitrozimtsäureester IV anschließend zur Aminoverbindung V reduziert und V mit einem Tetrahydrophthalsäureanhydrid kondensiert.

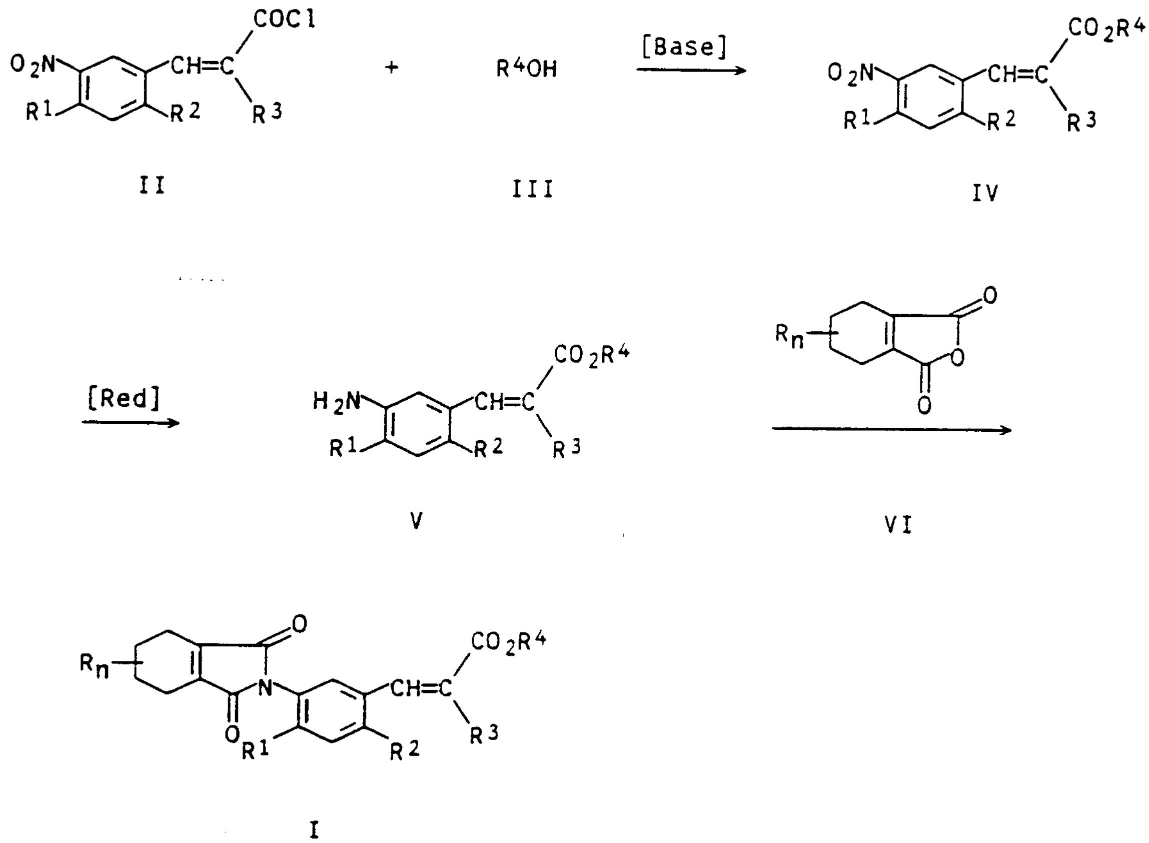


II III IV

V VI

I

Als Lösungsmittel für die Umsetzung von II mit III dienen vornehmlich höhersiedende Kohlenwasserstoffe wie Xylol und Toluol, Carbonsäureester wie Essigsäureethylester und Ether wie Dioxan und Tetrahydrofuran.

Die Umsetzung erfolgt in der Regel bei Temperaturen von -10 °C bis 200 °C, vorzugsweise bei 0 bis 150 °C.

Geeignete Basen für diese Umsetzung sind beispielsweise tertiäre Amine wie Triethylamin und Pyridin oder anorganische Salze wie Natriumhydroxid, Kaliumhydroxid und Kaliumcarbonat.

Die Anilinderivate der Formel V sind in an sich bekannter Weise aus den entsprechenden Nitrophenylderivaten IV entweder durch Reduktion mit anorganischen Verbindungen wie Zinn-II-salzen oder Eisen oder, sofern $R^3$ nicht Brom bzw. Chlor bedeutet, durch katalytische Hydrierung an Metallkontakten wie Raney-Nickel, Palladium und Platin erhältlich.

Die Reduktion wird im allgemeinen in protisch polaren Lösungsmitteln wie Eisessig bei Temperaturen von 0 °C bis 150 °C, vorzugsweise 20 °C bis 100 °C, durchgeführt. Bei der katalytischen Hydrierung verwendet man beispielsweise Methanol oder Tetrahydrofuran als Lösungsmittel. Die Temperatur liegt dann bei 0 °C bis 100 °C und der Wasserstoffdruck bei 1 bis 100 atm.

Für die Kondensation des 3,4,5,6-Tetrahydrophthalsäureanhydrides der Formel VI mit einem 5-Aminozimtsäureester der Formel V dienen inerte organische Lösungsmittel wie niedere Alkansäuren wie Essigsäure, Propionsäure und Isobuttersäure, sowie die Ester dieser Säuren wie Essigsäureethylester, höhersiedende Kohlenwasserstoffe wie Toluol und Xylol und/oder Dimethylformamid. Die Umsetzung erfolgt in der Regel bei Temperaturen zwischen 25 °C und dem Siedepunkt der jeweiligen Reaktionsmischung, vorzugsweise zwischen 50 und 140 °C. Beim Arbeiten in einem aprotischen Lösungsmittel empfiehlt es sich, das Wasser fortlaufend zu entfernen.

Sofern $R^3$ Chlor oder Brom bedeutet, lassen sich die Verbindungen I auch herstellen, indem man einen Nitrozimtsäureester der Formel IVb in einem inerten organischen Lösungsmittel mit dem entsprechenden Halogen zum Nitrozimtsäureester der Formel IV halogeniert, und dieses anschließend wie vorstehend beschrieben weiterbehandelt.

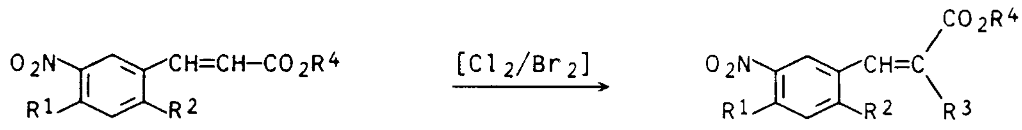


IVb

IV ($R^3$ = Cl, Br)

Die Halogenierung wird in der Regel bei Temperaturen von 0 bis 60 °C, vorzugsweise 15 bis 40 °C, durchgeführt.

Geeignete Lösungsmittel sind beispielsweise Methylenchlorid, Chloroform, Tetrachlorkohlenstoff, 1,1,1-Trichlorethan und Chlorbenzol, wobei Methylenchlorid, Chloroform und 1,1,1-Trichlorethan bevorzugt werden.

Man erhält die Verbindungen der Formel I aber auch, indem man einen geeignet substituierten Aldehyd IX mit einem Ylid der Formel X in an sich bekannter Weise, z. B. nach den in Synthesis (10), 862 (1984) beschriebenen Bedingungen, in einem Lösungsmittel bei Temperaturen zwischen Raumtemperatur und dem Siedepunkt des Lösungsmittels umsetzt.

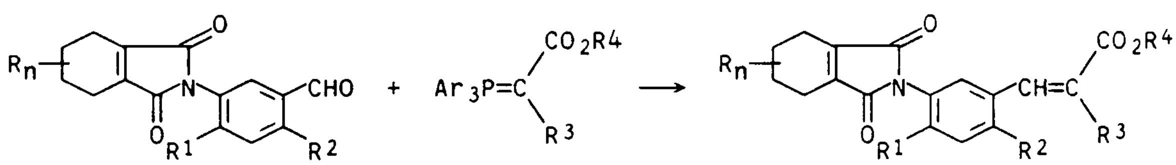

IX

X

I

Ar in Formel X bedeutet einen umsubstituierten oder substituierten Arylrest, wobei im allgemeinen der Phenylrest bevorzugt ist.

Als Lösungsmittel eignen sich z. B. Toluol, Tetrahydrofuran, Dimethylformamid, Dimethylsulfoxid und Methanol.

Die benötigten Triarylphosphorane X sind analog zu in der Literatur beschriebenen Verfahren zugänglich (Chem. Ber. 95 (1962) 3003).

Den benötigten Aldehyd IX erhält man beispielsweise indem man das Anilin VIII mit dem Anhydrid VI kondensiert. Verwendet man hierbei niedere Carbonsäuren, wie z. B. Essig- oder Propionsäure, spaltet gleichzeitig das Acetal.

Im Hinblick auf die bestimmungsgemäße Verwendung der Verbindungen I als Herbizide kommen als Substituenten vorzugsweise folgende Reste in Betracht:

$R^1$

Wasserstoff und Fluor,

$R^2$

Halogen wie Fluor, Chlor und Brom, insbesondere Chlor,

$R^3$

Wasserstoff; ein Halogenatom wie unter $R^2$ genannt, insbesondere Chlor und Brom; eine Alkylgruppe wie Methyl, Ethyl, Propyl, 1-Methylethyl, Butyl, 1-Methylpropyl, 2-Methylpropyl und 1,1-Dimethylethyl, insbesondere Methyl und Ethyl,

Wasserstoff, eine Alkylgruppe wie bei $R^3$ genannt sowie n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl und 1-Ethyl-2-methylpropyl, insbesondere Methyl, Ethyl, Propyl und iso-Propyl, wobei diese Alkylgruppe durch ein bis zwei $C_1$-$C_4$-Alkoxygruppen oder $C_1$-$C_4$-Alkylthiogruppen substituiert sein kann, wobei Alkoxyalkylgruppen wie Methoxymethyl, 2-Methoxyethyl, 2-Methoxypropyl, 3-Methoxypropyl, 2-Methoxy-1-methylethyl, Ethoxymethyl, 2-Ethoxyethyl, 2-Ethoxypropyl, 3-Ethoxypropyl, 2-Ethoxyl-methylethyl und 1-Ethoxy-1-methylethyl, insbesondere Methoxyethyl und Ethoxyethyl bedeuten und Alkylthioalkylgruppen insbesondere Methylthioethyl und Ethylthioethyl bedeuten;

eine Cycloalkylgruppe wie Cyclopropyl, Cyclobutyl, Cyclopentyl, Cyclohexyl und Cycloheptyl;
eine Alkenylgruppe wie 2-Propenyl, 2-Butenyl, 3-Butenyl, 1-Methyl-2-propenyl, 2-Methyl-2-propenyl, 2-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,2-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, oder eine entsprechende Alkenyloxygruppe,
eine Alkinylgruppe wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, oder eine entsprechende Alkinyloxygruppe, oder ein Benzylrest.

Beispiele für sehr aktive Verbindungen I sind in den nachstehenden Tabellen A und B aufgeführt.

Tabelle A:

Ia

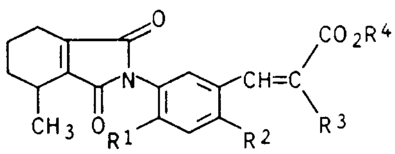

| $R^1$ | $R^2$ | $R^3$ | $R^4$ |
|---|---|---|---|
| H | Cl | H | H |
| F | Cl | H | H |
| H | Br | H | H |
| F | Br | H | H |
| H | Cl | Cl | H |
| F | Cl | Cl | H |
| H | Br | Cl | H |
| F | Br | Cl | H |
| H | Cl | Br | H |
| F | Cl | Br | H |
| H | Br | Br | H |
| F | Br | Br | H |
| H | Cl | $CH_3$ | H |
| F | Cl | $CH_3$ | H |
| H | Br | $CH_3$ | H |
| F | Br | $CH_3$ | H |
| H | Cl | $CH_2CH_3$ | H |
| F | Cl | $CH_2CH_3$ | H |
| H | Br | $CH_2CH_3$ | H |
| F | Br | $CH_2CH_3$ | H |
| H | Cl | H | $CH_3$ |
| F | Cl | H | $CH_3$ |
| H | Br | H | $CH_3$ |
| F | Br | H | $CH_3$ |
| H | Cl | Cl | $CH_3$ |
| F | Cl | Cl | $CH_3$ |

Tabelle A (Fortsetzung)

| $R^1$ | $R^2$ | $R^3$ | $R^4$ |
|---|---|---|---|
| H | Br | Cl | $CH_3$ |
| F | Br | Cl | $CH_3$ |
| H | Cl | Br | $CH_3$ |
| F | Cl | Br | $CH_3$ |
| H | Br | Br | $CH_3$ |
| F | Br | Br | $CH_3$ |
| H | Cl | $CH_3$ | $CH_3$ |
| F | Cl | $CH_3$ | $CH_3$ |
| H | Br | $CH_3$ | $CH_3$ |
| F | Br | $CH_3$ | $CH_3$ |
| H | Cl | $CH_2CH_3$ | $CH_3$ |
| F | Cl | $CH_2CH_3$ | $CH_3$ |
| H | Br | $CH_2CH_3$ | $CH_3$ |
| F | Br | $CH_2CH_3$ | $CH_3$ |
| H | Cl | H | $CH_2CH_3$ |
| F | Cl | H | $CH_2CH_3$ |
| H | Br | H | $CH_2CH_3$ |
| F | Br | H | $CH_2CH_3$ |
| H | Cl | Cl | $CH_2CH_3$ |
| F | Cl | Cl | $CH_2CH_3$ |
| H | Br | Cl | $CH_2CH_3$ |
| F | Br | Cl | $CH_2CH_3$ |
| H | Cl | Br | $CH_2CH_3$ |
| F | Cl | Br | $CH_2CH_3$ |
| H | Br | Br | $CH_2CH_3$ |
| F | Br | Br | $CH_2CH_3$ |
| H | Cl | $CH_3$ | $CH_2CH_3$ |
| F | Cl | $CH_3$ | $CH_2CH_3$ |
| H | Br | $CH_3$ | $CH_2CH_3$ |
| F | Br | $CH_3$ | $CH_2CH_3$ |
| H | Cl | $CH_2CH_3$ | $CH_2CH_3$ |
| F | Cl | $CH_2CH_3$ | $CH_2CH_3$ |
| H | Br | $CH_2CH_3$ | $CH_2CH_3$ |
| F | Br | $CH_2CH_3$ | $CH_2CH_3$ |
| H | Cl | H | $(CH_2)_2CH_3$ |
| F | Cl | H | $(CH_2)_2CH_3$ |
| H | Cl | Cl | $(CH_2)_2CH_3$ |
| F | Cl | Cl | $(CH_2)_2CH_3$ |

Tabelle A (Fortsetzung)

| $R^1$ | $R^2$ | $R^3$ | $R^4$ |
|---|---|---|---|
| H | Cl | Br | $(CH_2)_2CH_3$ |
| F | Cl | Br | $(CH_2)_2CH_3$ |
| H | Cl | $CH_3$ | $(CH_2)_2CH_3$ |
| F | Cl | $CH_3$ | $(CH_2)_2CH_3$ |
| H | Cl | $CH_2CH_3$ | $(CH_2)_2CH_3$ |
| F | Cl | $CH_2CH_3$ | $(CH_2)_2CH_3$ |
| H | Cl | H | $CH(CH_3)_2$ |
| F | Cl | H | $CH(CH_3)_2$ |
| H | Cl | Cl | $CH(CH_3)_2$ |
| F | Cl | Cl | $CH(CH_3)_2$ |
| H | Cl | Br | $CH(CH_3)_2$ |
| F | Cl | Br | $CH(CH_3)_2$ |
| H | Cl | $CH_3$ | $CH(CH_3)_2$ |
| F | Cl | $CH_3$ | $CH(CH_3)_2$ |
| H | Cl | $CH_2CH_3$ | $CH(CH_3)_2$ |
| F | Cl | $CH_2CH_3$ | $CH(CH_3)_2$ |
| H | Cl | H | $(CH_2)_3CH_3$ |
| F | Cl | H | $(CH_2)_3CH_3$ |
| H | Cl | Cl | $(CH_2)_3CH_3$ |
| F | Cl | Cl | $(CH_2)_3CH_3$ |
| H | Cl | Br | $(CH_2)_3CH_3$ |
| F | Cl | Br | $(CH_2)_3CH_3$ |
| H | Cl | $CH_3$ | $(CH_2)_3CH_3$ |
| F | Cl | $CH_3$ | $(CH_2)_3CH_3$ |
| H | Cl | $CH_2CH_3$ | $(CH_2)_3CH_3$ |
| F | Cl | $CH_2CH_3$ | $(CH_2)_3CH_3$ |
| H | Cl | H | $CH_2CH(CH_3)_2$ |
| F | Cl | H | $CH_2CH(CH_3)_2$ |
| H | Cl | Cl | $CH_2CH(CH_3)_2$ |
| F | Cl | Cl | $CH_2CH(CH_3)_2$ |
| H | Cl | Br | $CH_2CH(CH_3)_2$ |
| F | Cl | Br | $CH_2CH(CH_3)_2$ |
| H | Cl | $CH_3$ | $CH_2CH(CH_3)_2$ |
| F | Cl | $CH_3$ | $CH_2CH(CH_3)_2$ |
| H | Cl | $CH_2CH_3$ | $CH_2CH(CH_3)_2$ |
| F | Cl | $CH_2CH_3$ | $CH_2CH(CH_3)_2$ |
| H | Cl | H | $(CH_2)_4CH_3$ |
| F | Cl | H | $(CH_2)_4CH_3$ |

Tabelle A (Fortsetzung)

| $R^1$ | $R^2$ | $R^3$ | $R^4$ |
|---|---|---|---|
| H | Cl | Cl | $(CH_2)_4CH_3$ |
| F | Cl | Cl | $(CH_2)_4CH_3$ |
| H | Cl | Br | $(CH_2)_4CH_3$ |
| F | Cl | Br | $(CH_2)_4CH_3$ |
| H | Cl | $CH_3$ | $(CH_2)_4CH_3$ |
| F | Cl | $CH_3$ | $(CH_2)_4CH_3$ |
| H | Cl | $CH_2CH_3$ | $(CH_2)_4CH_3$ |
| F | Cl | $CH_2CH_3$ | $(CH_2)_4CH_3$ |
| H | Cl | H | $(CH_2)_2CH(CH_3)_2$ |
| F | Cl | H | $(CH_2)_2CH(CH_3)_2$ |
| H | Cl | Cl | $(CH_2)_2CH(CH_3)_2$ |
| F | Cl | Cl | $(CH_2)_2CH(CH_3)_2$ |
| H | Cl | Br | $(CH_2)_2CH(CH_3)_2$ |
| F | Cl | Br | $(CH_2)_2CH(CH_3)_2$ |
| H | Cl | $CH_3$ | $(CH_2)_2CH(CH_3)_2$ |
| F | Cl | $CH_3$ | $(CH_2)_2CH(CH_3)_2$ |
| H | Cl | $CH_2CH_3$ | $(CH_2)_2CH(CH_3)_2$ |
| F | Cl | $CH_2CH_3$ | $(CH_2)_2CH(CH_3)_2$ |
| H | Cl | H | $(CH_2)_2OCH_3$ |
| F | Cl | H | $(CH_2)_2OCH_3$ |
| H | Cl | Cl | $(CH_2)_2OCH_3$ |
| F | Cl | Cl | $(CH_2)_2OCH_3$ |
| H | Cl | Br | $(CH_2)_2OCH_3$ |
| F | Cl | Br | $(CH_2)_2OCH_3$ |
| H | Cl | $CH_3$ | $(CH_2)_2OCH_3$ |
| F | Cl | $CH_3$ | $(CH_2)_2OCH_3$ |
| H | Cl | $CH_2CH_3$ | $(CH_2)_2OCH_3$ |
| F | Cl | $CH_2CH_3$ | $(CH_2)_2OCH_3$ |
| H | Cl | H | $CH(CH_3)CH_2OCH_3$ |
| F | Cl | H | $CH(CH_3)CH_2OCH_3$ |
| H | Cl | Cl | $CH(CH_3)CH_2OCH_3$ |
| F | Cl | Cl | $CH(CH_3)CH_2OCH_3$ |
| H | Cl | Br | $CH(CH_3)CH_2OCH_3$ |
| F | Cl | Br | $CH(CH_3)CH_2OCH_3$ |
| H | Cl | $CH_3$ | $CH(CH_3)CH_2OCH_3$ |
| F | Cl | $CH_3$ | $CH(CH_3)CH_2OCH_3$ |
| H | Cl | $CH_2CH_3$ | $CH(CH_3)CH_2OCH_3$ |
| F | Cl | $CH_2CH_3$ | $CH(CH_3)CH_2OCH_3$ |

Tabelle A (Fortsetzung)

| $R^1$ | $R^2$ | $R^3$ | $R^4$ |
|---|---|---|---|
| H | Cl | H | $CH_2CH{=}CH_2$ |
| F | Cl | H | $CH_2CH{=}CH_2$ |
| H | Cl | Cl | $CH_2CH{=}CH_2$ |
| F | Cl | Cl | $CH_2CH{=}CH_2$ |
| H | Cl | Br | $CH_2CH{=}CH_2$ |
| F | Cl | Br | $CH_2CH{=}CH_2$ |
| H | Cl | $CH_3$ | $CH_2CH{=}CH_2$ |
| F | Cl | $CH_3$ | $CH_2CH{=}CH_2$ |
| H | Cl | $CH_2CH_3$ | $CH_2CH{=}CH_2$ |
| F | Cl | $CH_2CH_3$ | $CH_2CH{=}CH_2$ |
| H | Cl | H | $CH_2CH{=}CHCH_3$ |
| F | Cl | H | $CH_2CH{=}CHCH_3$ |
| H | Cl | Cl | $CH_2CH{=}CHCH_3$ |
| F | Cl | Cl | $CH_2CH{=}CHCH_3$ |
| H | Cl | Br | $CH_2CH{=}CHCH_3$ |
| F | Cl | Br | $CH_2CH{=}CHCH_3$ |
| H | Cl | $CH_3$ | $CH_2CH{=}CHCH_3$ |
| F | Cl | $CH_3$ | $CH_2CH{=}CHCH_3$ |
| H | Cl | $CH_2CH_3$ | $CH_2CH{=}CHCH_3$ |
| F | Cl | $CH_2CH_3$ | $CH_2CH{=}CHCH_3$ |
| H | Cl | H | $CH_2C{\equiv}CH$ |
| F | Cl | H | $CH_2C{\equiv}CH$ |
| H | Cl | Cl | $CH_2C{\equiv}CH$ |
| F | Cl | Cl | $CH_2C{\equiv}CH$ |
| H | Cl | Br | $CH_2C{\equiv}CH$ |
| F | Cl | Br | $CH_2C{\equiv}CH$ |
| H | Cl | $CH_3$ | $CH_2C{\equiv}CH$ |
| F | Cl | $CH_3$ | $CH_2C{\equiv}CH$ |
| H | Cl | $CH_2CH_3$ | $CH_2C{\equiv}CH$ |
| F | Cl | $CH_2CH_3$ | $CH_2C{\equiv}CH$ |
| H | Cl | H | $CH_2C{\equiv}CCH_3$ |
| F | Cl | H | $CH_2C{\equiv}CCH_3$ |
| H | Cl | Cl | $CH_2C{\equiv}CCH_3$ |
| F | Cl | Cl | $CH_2C{\equiv}CCH_3$ |
| H | Cl | Br | $CH_2C{\equiv}CCH_3$ |
| F | Cl | Br | $CH_2C{\equiv}CCH_3$ |
| H | Cl | $CH_3$ | $CH_2C{\equiv}CCH_3$ |
| F | Cl | $CH_3$ | $CH_2C{\equiv}CCH_3$ |

Tabelle A (Fortsetzung)

| $R^1$ | $R^2$ | $R^3$ | $R^4$ |
|---|---|---|---|
| H | Cl | $CH_2CH_3$ | $CH_2C{\equiv}CCH_3$ |
| F | Cl | $CH_2CH_3$ | $CH_2C{\equiv}CCH_3$ |
| H | Cl | H | $CH_2Ph$ |
| F | Cl | H | $CH_2Ph$ |
| H | Cl | Cl | $CH_2Ph$ |
| F | Cl | Cl | $CH_2Ph$ |
| H | Cl | Br | $CH_2Ph$ |
| F | Cl | Br | $CH_2Ph$ |
| H | Cl | $CH_3$ | $CH_2Ph$ |
| F | Cl | $CH_3$ | $CH_2Ph$ |
| H | Cl | $CH_2CH_3$ | $CH_2Ph$ |
| F | Cl | $CH_2CH_3$ | $CH_2Ph$ |
| H | F | Cl | $CH_3$ |
| H | F | Br | $CH_3$ |
| H | F | $CH_3$ | $CH_3$ |
| H | F | Cl | $CH_2CH_3$ |
| H | F | Br | $CH_2CH_3$ |
| H | F | $CH_3$ | $CH_2CH_3$ |
| H | F | Cl | $(CH_2)_2CH_3$ |
| H | F | Br | $(CH_2)_2CH_3$ |
| H | F | $CH_3$ | $(CH_2)_2CH_3$ |
| H | F | Cl | $CH(CH_3)_2$ |
| H | F | Br | $CH(CH_3)_2$ |
| H | F | $CH_3$ | $CH(CH_3)_2$ |
| H | F | Cl | $(CH_2)_3CH_3$ |
| H | F | Br | $(CH_2)_3CH_3$ |
| H | F | $CH_3$ | $(CH_2)_3CH_3$ |
| H | F | Cl | $CH_2CH(CH_3)_2$ |
| H | F | Br | $CH_2CH(CH_3)_2$ |
| H | F | $CH_3$ | $CH_2CH(CH_3)_2$ |
| H | F | Cl | $(CH_2)_4CH_3$ |
| H | F | Br | $(CH_2)_4CH_3$ |
| H | F | $CH_3$ | $(CH_2)_4CH_3$ |
| H | F | Cl | $(CH_2)_2CH(CH_3)_2$ |
| H | F | Br | $(CH_2)_2CH(CH_3)_2$ |
| H | F | $CH_3$ | $(CH_2)_2CH(CH_3)_2$ |
| H | F | Cl | $(CH_2)_2OCH_3$ |
| H | F | Br | $(CH_2)_2OCH_3$ |

Tabelle A (Fortsetzung)

| $R^1$ | $R^2$ | $R^3$ | $R^4$ |
|---|---|---|---|
| H | F | $CH_3$ | $(CH_2)_2OCH_3$ |
| H | F | Cl | $CH(CH_3)CH_2OCH_3$ |
| H | F | Br | $CH(CH_3)CH_2OCH_3$ |
| H | F | $CH_3$ | $CH(CH_3)CH_2OCH_3$ |
| H | F | Cl | $CH_2CH=CH_2$ |
| H | F | Br | $CH_2CH=CH_2$ |
| H | F | $CH_3$ | $CH_2CH=CH_2$ |
| H | F | Cl | $CH_2CH=CHCH_3$ |
| H | F | Br | $CH_2CH=CHCH_3$ |
| H | F | $CH_3$ | $CH_2CH=CHCH_3$ |
| H | F | Cl | $CH_2CH\equiv CH$ |
| H | F | Br | $CH_2CH\equiv CH$ |
| H | F | $CH_3$ | $CH_2CH\equiv CH$ |
| H | F | Cl | $CH_2C\equiv CCH_3$ |
| H | F | Br | $CH_2C\equiv CCH_3$ |
| H | F | $CH_3$ | $CH_2C\equiv CCH_3$ |
| H | F | Cl | $CH_2Ph$ |
| H | F | Br | $CH_2Ph$ |
| H | F | $CH_3$ | $CH_2Ph$ |

Tabelle B:

Ib

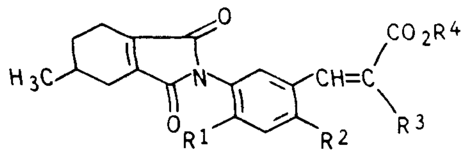

| $R^1$ | $R^2$ | $R^3$ | $R^4$ |
|---|---|---|---|
| H | Cl | H | H |
| F | Cl | H | H |
| H | Br | H | H |
| F | Br | H | H |
| H | Cl | Cl | H |
| F | Cl | Cl | H |
| H | Br | Cl | H |
| F | Br | Cl | H |
| H | Cl | Br | H |
| F | Cl | Br | H |
| H | Br | Br | H |
| F | Br | Br | H |
| H | Cl | $CH_3$ | H |
| F | Cl | $CH_3$ | H |
| H | Br | $CH_3$ | H |
| F | Br | $CH_3$ | H |
| H | Cl | $CH_2CH_3$ | H |
| F | Cl | $CH_2CH_3$ | H |
| H | Br | $CH_2CH_3$ | H |
| F | Br | $CH_2CH_3$ | H |
| H | Cl | H | $CH_3$ |
| F | Cl | H | $CH_3$ |
| H | Br | H | $CH_3$ |
| F | Br | H | $CH_3$ |
| H | Cl | Cl | $CH_3$ |
| F | Cl | Cl | $CH_3$ |

Tabelle B (Fortsetzung)

| $R^1$ | $R^2$ | $R^3$ | $R^4$ |
|---|---|---|---|
| H | Br | Cl | $CH_3$ |
| F | Br | Cl | $CH_3$ |
| H | Cl | Br | $CH_3$ |
| F | Cl | Br | $CH_3$ |
| H | Br | Br | $CH_3$ |
| F | Br | Br | $CH_3$ |
| H | Cl | $CH_3$ | $CH_3$ |
| F | Cl | $CH_3$ | $CH_3$ |
| H | Br | $CH_3$ | $CH_3$ |
| F | Br | $CH_3$ | $CH_3$ |
| H | Cl | $CH_2CH_3$ | $CH_3$ |
| F | Cl | $CH_2CH_3$ | $CH_3$ |
| H | Br | $CH_2CH_3$ | $CH_3$ |
| F | Br | $CH_2CH_3$ | $CH_3$ |
| H | Cl | H | $CH_2CH_3$ |
| F | Cl | H | $CH_2CH_3$ |
| H | Br | H | $CH_2CH_3$ |
| F | Br | H | $CH_2CH_3$ |
| H | Cl | Cl | $CH_2CH_3$ |
| F | Cl | Cl | $CH_2CH_3$ |
| H | Br | Cl | $CH_2CH_3$ |
| F | Br | Cl | $CH_2CH_3$ |
| H | Cl | Br | $CH_2CH_3$ |
| F | Cl | Br | $CH_2CH_3$ |
| H | Br | Br | $CH_2CH_3$ |
| F | Br | Br | $CH_2CH_3$ |
| H | Cl | $CH_3$ | $CH_2CH_3$ |
| F | Cl | $CH_3$ | $CH_2CH_3$ |
| H | Br | $CH_3$ | $CH_2CH_3$ |
| F | Br | $CH_3$ | $CH_2CH_3$ |
| H | Cl | $CH_2CH_3$ | $CH_2CH_3$ |
| F | Cl | $CH_2CH_3$ | $CH_2CH_3$ |
| H | Br | $CH_2CH_3$ | $CH_2CH_3$ |
| F | Br | $CH_2CH_3$ | $CH_2CH_3$ |
| H | Cl | H | $(CH_2)_2CH_3$ |
| F | Cl | H | $(CH_2)_2CH_3$ |
| H | Cl | Cl | $(CH_2)_2CH_3$ |
| F | Cl | Cl | $(CH_2)_2CH_3$ |

Tabelle B (Fortsetzung)

| $R^1$ | $R^2$ | $R^3$ | $R^4$ |
|---|---|---|---|
| H | Cl | Br | $(CH_2)_2CH_3$ |
| F | Cl | Br | $(CH_2)_2CH_3$ |
| H | Cl | $CH_3$ | $(CH_2)_2CH_3$ |
| F | Cl | $CH_3$ | $(CH_2)_2CH_3$ |
| H | Cl | $CH_2CH_3$ | $(CH_2)_2CH_3$ |
| F | Cl | $CH_2CH_3$ | $(CH_2)_2CH_3$ |
| H | Cl | H | $CH(CH_3)_2$ |
| F | Cl | H | $CH(CH_3)_2$ |
| H | Cl | Cl | $CH(CH_3)_2$ |
| F | Cl | Cl | $CH(CH_3)_2$ |
| H | Cl | Br | $CH(CH_3)_2$ |
| F | Cl | Br | $CH(CH_3)_2$ |
| H | Cl | $CH_3$ | $CH(CH_3)_2$ |
| F | Cl | $CH_3$ | $CH(CH_3)_2$ |
| H | Cl | $CH_2CH_3$ | $CH(CH_3)_2$ |
| F | Cl | $CH_2CH_3$ | $CH(CH_3)_2$ |
| H | Cl | H | $(CH_2)_3CH_3$ |
| F | Cl | H | $(CH_2)_3CH_3$ |
| H | Cl | Cl | $(CH_2)_3CH_3$ |
| F | Cl | Cl | $(CH_2)_3CH_3$ |
| H | Cl | Br | $(CH_2)_3CH_3$ |
| F | Cl | Br | $(CH_2)_3CH_3$ |
| H | Cl | $CH_3$ | $(CH_2)_3CH_3$ |
| F | Cl | $CH_3$ | $(CH_2)_3CH_3$ |
| H | Cl | $CH_2CH_3$ | $(CH_2)_3CH_3$ |
| F | Cl | $CH_2CH_3$ | $(CH_2)_3CH_3$ |
| H | Cl | H | $CH_2CH(CH_3)_2$ |
| F | Cl | H | $CH_2CH(CH_3)_2$ |
| H | Cl | Cl | $CH_2CH(CH_3)_2$ |
| F | Cl | Cl | $CH_2CH(CH_3)_2$ |
| H | Cl | Br | $CH_2CH(CH_3)_2$ |
| F | Cl | Br | $CH_2CH(CH_3)_2$ |
| H | Cl | $CH_3$ | $CH_2CH(CH_3)_2$ |
| F | Cl | $CH_3$ | $CH_2CH(CH_3)_2$ |
| H | Cl | $CH_2CH_3$ | $CH_2CH(CH_3)_2$ |
| F | Cl | $CH_2CH_3$ | $CH_2CH(CH_3)_2$ |
| H | Cl | H | $(CH_2)_4CH_3$ |
| F | Cl | H | $(CH_2)_4CH_3$ |

Tabelle B (Fortsetzung)

| $R^1$ | $R^2$ | $R^3$ | $R^4$ |
|---|---|---|---|
| H | Cl | Cl | $(CH_2)_4CH_3$ |
| F | Cl | Cl | $(CH_2)_4CH_3$ |
| H | Cl | Br | $(CH_2)_4CH_3$ |
| F | Cl | Br | $(CH_2)_4CH_3$ |
| H | Cl | $CH_3$ | $(CH_2)_4CH_3$ |
| F | Cl | $CH_3$ | $(CH_2)_4CH_3$ |
| H | Cl | $CH_2CH_3$ | $(CH_2)_4CH_3$ |
| F | Cl | $CH_2CH_3$ | $(CH_2)_4CH_3$ |
| H | Cl | H | $(CH_2)_2CH(CH_3)_2$ |
| F | Cl | H | $(CH_2)_2CH(CH_3)_2$ |
| H | Cl | Cl | $(CH_2)_2CH(CH_3)_2$ |
| F | Cl | Cl | $(CH_2)_2CH(CH_3)_2$ |
| H | Cl | Br | $(CH_2)_2CH(CH_3)_2$ |
| F | Cl | Br | $(CH_2)_2CH(CH_3)_2$ |
| H | Cl | $CH_3$ | $(CH_2)_2CH(CH_3)_2$ |
| F | Cl | $CH_3$ | $(CH_2)_2CH(CH_3)_2$ |
| H | Cl | $CH_2CH_3$ | $(CH_2)_2CH(CH_3)_2$ |
| F | Cl | $CH_2CH_3$ | $(CH_2)_2CH(CH_3)_2$ |
| H | Cl | H | $(CH_2)_2OCH_3$ |
| F | Cl | H | $(CH_2)_2OCH_3$ |
| H | Cl | Cl | $(CH_2)_2OCH_3$ |
| F | Cl | Cl | $(CH_2)_2OCH_3$ |
| H | Cl | Br | $(CH_2)_2OCH_3$ |
| F | Cl | Br | $(CH_2)_2OCH_3$ |
| H | Cl | $CH_3$ | $(CH_2)_2OCH_3$ |
| F | Cl | $CH_3$ | $(CH_2)_2OCH_3$ |
| H | Cl | $CH_2CH_3$ | $(CH_2)_2OCH_3$ |
| F | Cl | $CH_2CH_3$ | $(CH_2)_2OCH_3$ |
| H | Cl | H | $CH(CH_3)CH_2OCH_3$ |
| F | Cl | H | $CH(CH_3)CH_2OCH_3$ |
| H | Cl | Cl | $CH(CH_3)CH_2OCH_3$ |
| F | Cl | Cl | $CH(CH_3)CH_2OCH_3$ |
| H | Cl | Br | $CH(CH_3)CH_2OCH_3$ |
| F | Cl | Br | $CH(CH_3)CH_2OCH_3$ |
| H | Cl | $CH_3$ | $CH(CH_3)CH_2OCH_3$ |
| F | Cl | $CH_3$ | $CH(CH_3)CH_2OCH_3$ |
| H | Cl | $CH_2CH_3$ | $CH(CH_3)CH_2OCH_3$ |
| F | Cl | $CH_2CH_3$ | $CH(CH_3)CH_2OCH_3$ |

Tabelle B (Fortsetzung)

| $R^1$ | $R^2$ | $R^3$ | $R^4$ |
|---|---|---|---|
| H | Cl | H | $CH_2CH=CH_2$ |
| F | Cl | H | $CH_2CH=CH_2$ |
| H | Cl | Cl | $CH_2CH=CH_2$ |
| F | Cl | Cl | $CH_2CH=CH_2$ |
| H | Cl | Br | $CH_2CH=CH_2$ |
| F | Cl | Br | $CH_2CH=CH_2$ |
| H | Cl | $CH_3$ | $CH_2CH=CH_2$ |
| F | Cl | $CH_3$ | $CH_2CH=CH_2$ |
| H | Cl | $CH_2CH_3$ | $CH_2CH=CH_2$ |
| F | Cl | $CH_2CH_3$ | $CH_2CH=CH_2$ |
| H | Cl | H | $CH_2CH=CHCH_3$ |
| F | Cl | H | $CH_2CH=CHCH_3$ |
| H | Cl | Cl | $CH_2CH=CHCH_3$ |
| F | Cl | Cl | $CH_2CH=CHCH_3$ |
| H | Cl | Br | $CH_2CH=CHCH_3$ |
| F | Cl | Br | $CH_2CH=CHCH_3$ |
| H | Cl | $CH_3$ | $CH_2CH=CHCH_3$ |
| F | Cl | $CH_3$ | $CH_2CH=CHCH_3$ |
| H | Cl | $CH_2CH_3$ | $CH_2CH=CHCH_3$ |
| F | Cl | $CH_2CH_3$ | $CH_2CH=CHCH_3$ |
| H | Cl | H | $CH_2C\equiv CH$ |
| F | Cl | H | $CH_2C\equiv CH$ |
| H | Cl | Cl | $CH_2C\equiv CH$ |
| F | Cl | Cl | $CH_2C\equiv CH$ |
| H | Cl | Br | $CH_2C\equiv CH$ |
| F | Cl | Br | $CH_2C\equiv CH$ |
| H | Cl | $CH_3$ | $CH_2C\equiv CH$ |
| F | Cl | $CH_3$ | $CH_2C\equiv CH$ |
| H | Cl | $CH_2CH_3$ | $CH_2C\equiv CH$ |
| F | Cl | $CH_2CH_3$ | $CH_2C\equiv CH$ |
| H | Cl | H | $CH_2C\equiv CCH_3$ |
| F | Cl | H | $CH_2C\equiv CCH_3$ |
| H | Cl | Cl | $CH_2C\equiv CCH_3$ |
| F | Cl | Cl | $CH_2C\equiv CCH_3$ |
| H | Cl | Br | $CH_2C\equiv CCH_3$ |
| F | Cl | Br | $CH_2C\equiv CCH_3$ |
| H | Cl | $CH_3$ | $CH_2C\equiv CCH_3$ |
| F | Cl | $CH_3$ | $CH_2C\equiv CCH_3$ |

Tabelle B (Fortsetzung)

| $R^1$ | $R^2$ | $R^3$ | $R^4$ |
|---|---|---|---|
| H | Cl | $CH_2CH_3$ | $CH_2C{\equiv}CCH_3$ |
| F | Cl | $CH_2CH_3$ | $CH_2C{\equiv}CCH_3$ |
| H | Cl | H | $CH_2Ph$ |
| F | Cl | H | $CH_2Ph$ |
| H | Cl | Cl | $CH_2Ph$ |
| F | Cl | Cl | $CH_2Ph$ |
| H | Cl | Br | $CH_2Ph$ |
| F | Cl | Br | $CH_2Ph$ |
| H | Cl | $CH_3$ | $CH_2Ph$ |
| F | Cl | $CH_3$ | $CH_2Ph$ |
| H | Cl | $CH_2CH_3$ | $CH_2Ph$ |
| F | Cl | $CH_2CH_3$ | $CH_2Ph$ |
| H | F | Cl | $CH_3$ |
| H | F | Br | $CH_3$ |
| H | F | $CH_3$ | $CH_3$ |
| H | F | Cl | $CH_2CH_3$ |
| H | F | Br | $CH_2CH_3$ |
| H | F | $CH_3$ | $CH_2CH_3$ |
| H | F | Cl | $(CH_2)_2CH_3$ |
| H | F | Br | $(CH_2)_2CH_3$ |
| H | F | $CH_3$ | $(CH_2)_2CH_3$ |
| H | F | Cl | $CH(CH_3)_2$ |
| H | F | Br | $CH(CH_3)_2$ |
| H | F | $CH_3$ | $CH(CH_3)_2$ |
| H | F | Cl | $(CH_2)_3CH_3$ |
| H | F | Br | $(CH_2)_3CH_3$ |
| H | F | $CH_3$ | $(CH_2)_3CH_3$ |
| H | F | Cl | $CH_2CH(CH_3)_2$ |
| H | F | Br | $CH_2CH(CH_3)_2$ |
| H | F | $CH_3$ | $CH_2CH(CH_3)_2$ |
| H | F | Cl | $(CH_2)_4CH_3$ |
| H | F | Br | $(CH_2)_4CH_3$ |
| H | F | $CH_3$ | $(CH_2)_4CH_3$ |
| H | F | Cl | $(CH_2)_2CH(CH_3)_2$ |
| H | F | Br | $(CH_2)_2CH(CH_3)_2$ |
| H | F | $CH_3$ | $(CH_2)_2CH(CH_3)_2$ |
| H | F | Cl | $(CH_2)_2OCH_3$ |
| H | F | Br | $(CH_2)_2OCH_3$ |

Tabelle B (Fortsetzung)

| $R^1$ | $R^2$ | $R^3$ | $R^4$ |
|---|---|---|---|
| H | F | $CH_3$ | $(CH_2)_2OCH_3$ |
| H | F | Cl | $CH(CH_3)CH_2OCH_3$ |
| H | F | Br | $CH(CH_3)CH_2OCH_3$ |
| H | F | $CH_3$ | $CH(CH_3)CH_2OCH_3$ |
| H | F | Cl | $CH_2CH{=}CH_2$ |
| H | F | Br | $CH_2CH{=}CH_2$ |
| H | F | $CH_3$ | $CH_2CH{=}CH_2$ |
| H | F | Cl | $CH_2CH{=}CHCH_3$ |
| H | F | Br | $CH_2CH{=}CHCH_3$ |
| H | F | $CH_3$ | $CH_2CH{=}CHCH_3$ |
| H | F | Cl | $CH_2CH{\equiv}CH$ |
| H | F | Br | $CH_2CH{\equiv}CH$ |
| H | F | $CH_3$ | $CH_2CH{\equiv}CH$ |
| H | F | Cl | $CH_2C{\equiv}CCH_3$ |
| H | F | Br | $CH_2C{\equiv}CCH_3$ |
| H | F | $CH_3$ | $CH_2C{\equiv}CCH_3$ |
| H | F | Cl | $CH_2Ph$ |
| H | F | Br | $CH_2Ph$ |
| H | F | $CH_3$ | $CH_2Ph$ |

Die 5-(N-3,4,5,6-tetrahydrophthalimido)-zimtsäureester I bzw. die sie enthaltenden herbiziden und entblätternden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:

I. Man vermischt 90 Gewichtsteile der Verbindung Nr. 1.001 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.004 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.003 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 1.005 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 1.004 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 1.001 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.004 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 1.002 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3, vorzugsweise 0,01 bis 1 kg/ha aktive Substanz (a.S.).

Die Verbindungen I können daneben zur Dessikation von Baumwolle eingesetzt werden.

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |

| Botanischer Name | Deutscher Name |
|---|---|
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |

| Botanischer Name | Deutscher Name |
|---|---|
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die 5-(N-3,4,5,6-tetrahydrophthalimido)zimtsäureester I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die neuen Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

Beispiel 1

Herstellung von E/Z-N-[3-(2-methoxycarbonylprop-1-enyl)-4-chlorphenyl]-4(R,S)-methyl-3,4,5 ,6-tetrahydrophthalimid

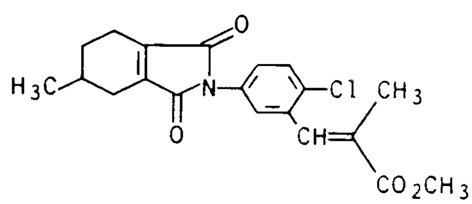

a) Eine Lösung von 468 g (1,8 mol) 2-Chlor-5-nitro-α-methylzimtsäurechlorid in 2250 ml Methanol wurden bei 5 °C mit 167,4 g (1,8 mol) α-Picolin versetzt. Die Reaktionsmischung rührte 12 Stunden bei 23 °C. Nach dem Abkühlen auf 0 °C wurde der entstandene Niederschlag isoliert und aus Methanol umkristallisiert. Man erhielt 294 g (64 %) 2-Chlor-5-nitro-α-methylzimtsäuremethylester (Fp. 95-96 °C).

b) Zu einer Mischung von 149 g Eisenpulver in 120 ml Eisessig wurden bei Rückfluß in 250 ml Methanol und 350 ml Eisessig heiß gelösten Nitrozimtsäureester (105 g; 0,4 mol) gegeben und 90 Minuten am Rückfluß erhitzt. Nach dem Abkühlen wurde abgesaugt, in Wasser aufgenommen und dreimal mit Essigester extrahiert, die organischen Phasen mit Wasser gewaschen, getrocknet und eingeengt. Man

erhielt 81,1 g (88 %) von 2-Chlor-5-amino-α-methylzimtsäuremethylester(Fp. 80 ° C).

c) 4,5 g (0,02 mol) des obigen Anilins und 3,5 g (0,021 mol) 4-Methyl-cyclohexen-1,2-dicarbonsäureanhydrid wurden in 120 ml Eisessig 17 Stunden bei 60 ° C gerührt, eingeengt und aus Methanol umkristallisiert. Man erhielt 4,7 g (63 %) der Titelverbindung (76-78 ° C). (Tabelle 1, Nr. 1.001)

Beispiel 2

Herstellung von E/Z-N-[3-(2-chlor-2-methoxycarbonylethen-1-yl)-4-chlorphenyl]-4(R,S)-methyl-3,4,5,6-tetrahydrophthalimid

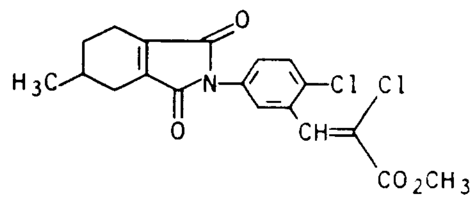

a) 29,9 2 (0,15 mol) 4-Chlor-3-(1,3-dioxolan-2-yl)-anilin und 24,9 g (0,15 mol) 4(R,S)-Methyl-cyclohexen-1,2-dicarbonsäureanhydrid wurden in 250 ml Eisessig 3 Tage bei 23 ° C und 10 Stunden bei 70 ° C gerührt, durch Zugabe von Wasser ausgefällt, umkristallisiert und getrocknet. Man erhielt 38 g (83 %) N-(4-Chlor-3-formyl-phenyl)-4(R,S)-methyl-3,4,5,6-tetrahydrophthalimid (Fp. 132-133 ° C).

b) 3 g (0,01 mol) des obigen Aldehyds und 3,7 g (0,01 mol) Carbomethoxychlormethylen-triphenylphosphoran in 50 ml Methanol wurden 5 Stunden bei 23 ° C gerührt. Nach Zugabe von 25 ml Wasser wurde der Niederschlag abgesaugt, mit Petrolether gewaschen und getrocknet. Man erhielt 3,0 g (76 %) der Titelverbindung (Fp. 91-92 ° C). Tabelle 1, Nr. 1.004).

**Tabelle 1**

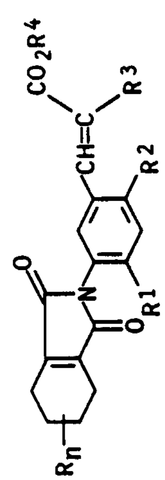

| Wirkstoff-Nr. | $R^1$ | $R^2$ | $R^3$ | $R^4$ | $(R)_n$ | physik. Daten Fp (°C); IR ($cm^{-1}$) |
|---|---|---|---|---|---|---|
| 1.001 | H | Cl | $CH_3$ | $CH_3$ | 4-$CH_3$ | 76-78 |
| 1.002 | H | Cl | Br | $CH_3$ | 4-$CH_3$ | 95-96 |
| 1.003 | H | Cl | Br | $CH_2CH_3$ | 4-$CH_3$ | 72-73 |
| 1.004 | H | Cl | Cl | $CH_3$ | 4-$CH_3$ | 91-92 |
| 1.005 | H | Cl | Cl | $CH_2CH_3$ | 4-$CH_3$ | 82-83 |
| 1.006 | H | Cl | $CH_3$ | $CH_2CH_3$ | 4-$CH_3$ | 1714,1474,1376,1255 |
| 1.007 | H | Cl | Br | $C(CH_3)_3$ | 4-$CH_3$ | 97-98 |
| 1.008 | H | Cl | Br | $CH(CH_3)_2$ | 4-$CH_3$ | 105-106 |
| 1.009 | H | Cl | Cl | $C(CH_3)_3$ | 4-$CH_3$ | 118-119 |
| 1.010 | H | Cl | Cl | $CH(CH_3)_2$ | 4-$CH_3$ | 120-121 |
| 1.011 | F | Cl | Br | $CH_3$ | 4-$CH_3$ | 125-126 |
| 1.012 | F | Cl | Cl | $CH_2CH_3$ | 4-$CH_3$ | 1721,1488,1419,1238 |
| 1.013 | F | Cl | Cl | $CH_3$ | 4-$CH_3$ | 1721,1489,1420,1241 |

Anwendungsbeispiele

Die herbizide Wirkung der 5-(N-3,4,5,6-tetrahydrophthalimido)-zimtsäureesterder Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies

nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,03 und 0,06 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und O keine Schädigung oder normaler Wachstumsverlauf.

Die in den Gewächshausversuchen verwendeten Pflanzen setzten sich aus folgenden Arten zusammen:

| Lateinischer Name | Deutscher Name |
|---|---|
| Abutilon theophrasti | Chinesischer Hanf |
| Amaranthus retroflexus | Zurückgekrümmter Fuchsschwanz |
| Chrysanthemum corinarium | Kronenwucherblume |
| Oryza sativa | Reis |
| Solanum nigrum | Schwarzer Nachtschatten |

Mit 0,03 bzw 0,06 kg/ha a.S. im Nachauflaufverfahren eingesetzt, lassen sich mit den Beispielen 1.001, 1.004 und 1.005 breitblättrige unerwünschte Pflanzen sehr gut bekämpfen, wobei Beispiel 1.001 gleichzeitig Verträglichkeit bei Reis zeigt.

**Patentansprüche**

**Patentansprüche für folgende Vertragsstaaten : AT, BE, CH, DE, DK, FR, GB, IT, LI, LU, NL, SE**

**1.** 5-(N-3,4,5,6-tetrahydrophthalimido)zimtsäureester der allgemeinen Formel I,

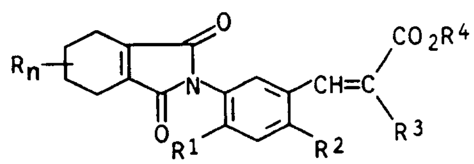

I

in der die Substituenten und der Index folgende Bedeutung haben:

- $R^1$ Wasserstoff oder Fluor,
- $R^2$ Halogen,
- $R^3$ Wasserstoff, Halogen oder eine $C_1$-$C_4$-Alkylgruppe,
- $R^4$ Wasserstoff, eine $C_1$-$C_6$-Alkylgruppe, welche durch ein oder zwei $C_1$-$C_4$-Alkoxy- oder $C_1$-$C_4$-Alkylthiogruppen substituiert sein kann, eine $C_3$-$C_7$-Cycloalkylgruppe, eine $C_3$-$C_6$-Alkenylgruppe, eine $C_3$-$C_6$-Alkinylgruppe oder ein Benzylrest,
- R $C_1$-$C_4$-Alkyl,
- n 1 oder 2.

**2.** Verfahren zur Herstellung der Verbindung I gemäß Anspruch 1, dadurch gekenneichnet, daß man ein Nitrozimtsäurechlorid der allgemeinen Formel II

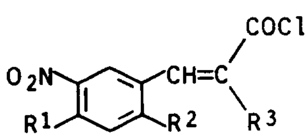

II

in an sich bekannter Weise in einem inerten organischen Lösungsmittel in Anwesenheit einer Base mit einem Alkohol der Formel III

$R^4OH$ III

zum Nitrozimtsäureester IV umsetzt,

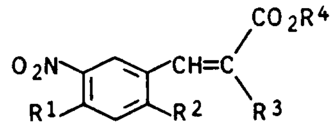 IV

den Nitrozimtsäureester IV anschließend zur Aminoverbindung V reduziert

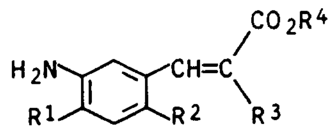 V

und V mit einem Tetrahydrophthalsäureanhydrid VI kondensiert.

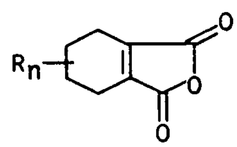 VI

**3.** Verfahren zur Herstellung der Verbindung I gemäß Anspruch 1, in der $R^3$ Chlor oder Brom bedeutet, dadurch gekennzeichnet, daß man einen Nitrozimtsäureester der Formel IVb

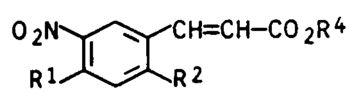 IVb

in einem inerten organischen Lösungsmittel mit Chlor oder Brom zum Nitrozimtsäureester der Formel IV

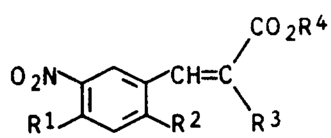 IV

halogeniert, anschließend zu V reduziert und mit dem Tetrahydrophthalsäureanhydrid VI gemäß Anspruch 2 kondensiert.

**4.** Verfahren zur Herstellung der Verbindungen I gemäß Anspruch 1, dadurch gekennzeichnet, daß man ein Zimtsäurechlorid der Formel VII

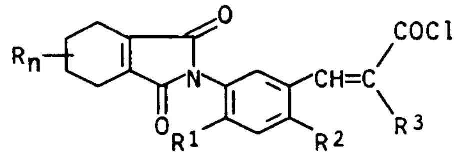 VII

in an sich bekannter Weise in einem inerten organischen Lösungsmittel mit einem Alkohol der Formel III gemäß Anspruch 2, zum Zimtsäureester I umsetzt.

**5.** Verfahren zur Herstellung der Verbindungen I gemäß Anspruch 1, dadurch gekennzeichnet, daß man ein Acetal der Formel VIII,

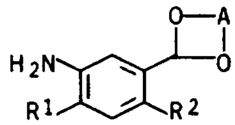

VIII

in der A eine Ethylen- oder Propyleneinheit bedeutet, welche durch ein bis drei $C_1$-$C_3$-Alkylgruppen substituiert sein kann, mit einem Tetrahydrophthalsäureanhydrid der Formel VI gemäß Anspruch 2 unter Abspaltung des Diols zum Aldehyd IX

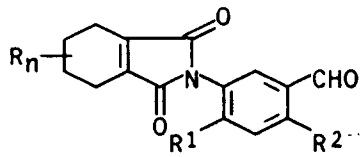

IX

kondensiert und IX anschließend mit einem Ylid der Formel X,

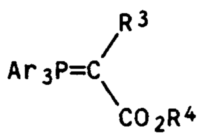

X

worin Ar einen Arylrest bedeutet, umsetzt.

**6.** Verwendung der 5-(N-3,4,5,6-tetrahydrophthalimido)zimtsäureester der Formel I gemäß Anspruch 1 als Herbizide.

**7.** Herbizide Mittel, enthaltend einen 5-(N-3,4,5,6-tetrahydrophthalimido)zimtsäureester der Formel I gemäß Anspruch 1 und inerte Zusatzstoffe.

**8.** Herbizide Mittel gemäß Anspruch 7, enthaltend ein 5-(N-3,4,5,6-tetrahydrophthalimido)zimtsäureester der Formel I und weitere wirksame Bestandteile.

**9.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines 5-(N-3,4,5,6-tetrahydrophthalimido)zimtsäureesters I gemäß Anspruch 1 behandelt.

**10.** Verfahren zur Entblätterung von Baumwolle, dadurch gekennzeichnet, daß man die Baumwollpflanzen mit einer wirksamen Menge eines 5-(N-3,4,5,6-tetrahydrophthalimido)zimtsäureesters I gemäß Anspruch 1 behandelt.

**Patentansprüche für folgenden Vertragsstaat : ES**

**1.** Herbizide Mittel enthaltend inerte Zusatzstoffe und einen 5-(N-3,4,5,6-tetrahydrophthalimido)-zimtsäureester der Formel I

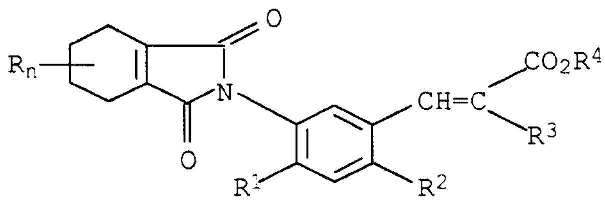

I

in der die Substituenten und der Index folgende Bedeutung haben:

- $R^1$ Wasserstoff oder Fluor,
- $R^2$ Halogen,
- $R^3$ Wasserstoff, Halogen oder eine $C_1$-$C_4$-Alkylgruppe,
- $R^4$ Wasserstoff, eine $C_1$-$C_6$-Alkylgruppe, welche durch ein oder zwei $C_1$-$C_4$-Alkoxy- oder $C_1$-$C_4$-Alkylthiogruppen substituiert sein kann, eine $C_3$-$C_7$-Cycloalkylgruppe, eine $C_3$-$C_6$-Alkenylgruppe, eine $C_3$-$C_6$-Alkinylgruppe oder den Benzylrest,
- R $C_1$-$C_4$-Alkyl,
- n 1 oder 2.

**2.** Herbizide Mittel nach Anspruch 1, enthaltend einen 5-(N-3,4,5,6-tetrahydrophthalimido)zimtsäureester der Formel I und weitere wirksame Bestandteile.

**3.** Verfahren zur Herstellung der Verbindung I gemäß Anspruch 1, dadurch gekennzeichnet, daß man ein Nitrozimtsäurechlorid der allgemeinen Formel II

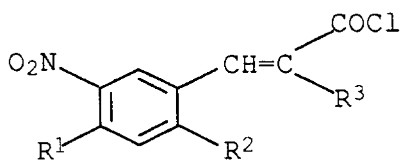

II

in an sich bekannter Weise in einem inerten organischen Lösungsmittel in Anwesenheit einer Base mit einem Alkohol der Formel III

$R^4OH$ III

zum Nitrozimtsäureester IV umsetzt,

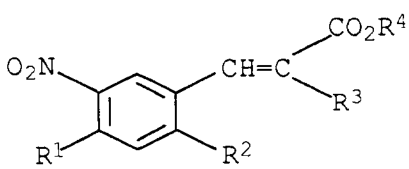

IV

den Nitrozimtsäureester IV anschließend zur Aminoverbindung V reduziert

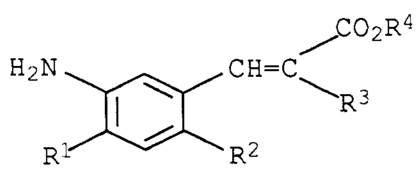

V

und V mit einem Tetrahydrophthalsäureanhydrid VI

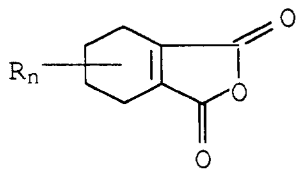

VI

kondensiert.

4. Verfahren zur Herstellung der Verbindung I gemäß Anspruch 1, in der $R^3$ Chlor oder Brom bedeutet, dadurch gekennzeichnet, daß man einen Nitrozimtsäureester der Formel IVb

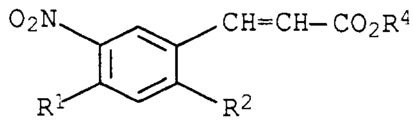

IVb

in einem inerten organischen Lösungsmittel mit Chlor oder Brom zum Nitrozimtsäureester der Formel IV

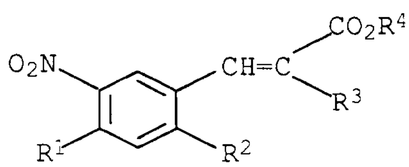

IV

halogeniert, anschließend zu V reduziert und mit dem Tetrahydrophthalsäureanhydrid VI gemäß Anspruch 3 kondensiert.

5. Verfahren zur Herstellung der Verbindungen I gemäß Anspruch 1, dadurch gekennzeichnet, daß man ein Zimtsäurechlorid der Formel VII

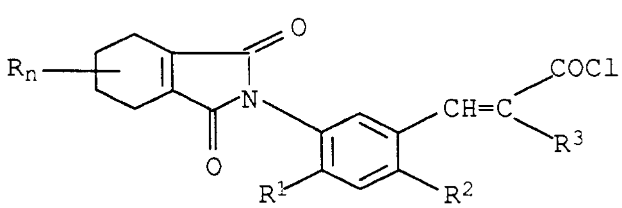

VII

in an sich bekannter Weise in einem inerten organischen Lösungsmittel mit einem Alkohol der Formel III gemäß Anspruch 3 zum Zimtsäureester I umsetzt.

6. Verfahren zur Herstellung der Verbindungen I gemäß Anspruch 1, dadurch gekennzeichnet, daß man ein Acetal der Formel VIII,

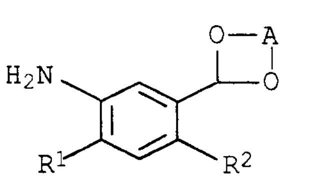

VIII

in der A eine Ethylen- oder Propyleneinheit bedeutet, welche durch ein bis drei $C_1$-$C_3$-Alkylgruppen substituiert sein kann, mit einem Tetrahydrophthalsäureanhydrid der Formel VI gemäß Anspruch 3 unter Abspaltung des Diols zum Aldehyd IX

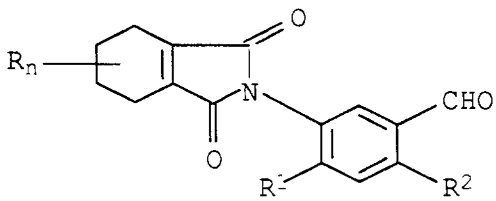

IX

kondensiert und IX anschließend mit einem Ylid der Formel X

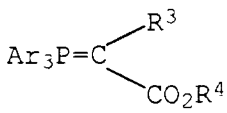

X,

worin Ar einen Arylrest bedeutet, umsetzt.

7. Verwendung der 5-(N-3,4,5,6-tetrahydrophthalimido)zimtsäureester der Formel I gemäß Anspruch 1 als Herbizide.

8. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines 5-(N-3,4,5,6-tetrahydrophthalimido)zimtsäureesters I gemäß Anspruch 1 behandelt.

9. Verfahren zur Entblätterung von Baumwolle, dadurch gekennzeichnet, daß man die Baumwollpflanzen mit einer wirksamen Menge eines 5-(N-3,4,5,6-tetrahydrophthalimido)zimtsäureesters I gemäß Anspruch 1 behandelt.

**Claims**

**Claims for the following Contracting States : AT, BE, CH, DE, DK, FR, GB, IT, LI, LU, NL, SE**

1. A 5-(N-3,4,5,6-tetrahydrophthalimido)cinnamic ester of the general formula I

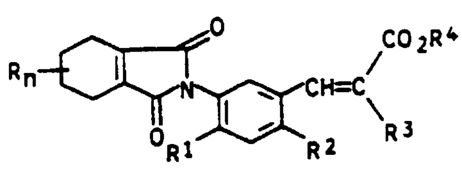

I,

where $R^1$ is hydrogen or fluorine, $R^2$ is halogen, $R^3$ is hydrogen, halogen or $C_1$-$C_4$-alkyl, $R^4$ is hydrogen, $C_1$-$C_6$-alkyl which can be substituted by one or two $C_1$-$C_4$-alkoxy or $C_1$-$C_4$-alkylthio groups, or $C_3$-$C_7$-cycloalkyl, $c_3$-$C_6$-alkenyl, $C_3$-$C_6$-alkynyl or benzyl, R is $C_1$-$C_4$-alkyl, and n is 1 or 2.

2. A process for the preparation of a compound I as claimed in claim 1, wherein a nitrocinnamyl chloride of the general formula II

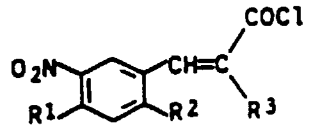

II

is reacted in a conventional manner in an inert organic solvent in the presence of a base with an alcohol of the formula III

$R^4OH$ III

to give the nitrocinnamic ester IV

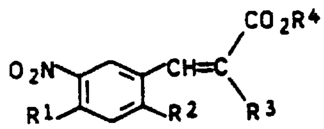

IV,

the nitrocinnamic ester IV is subsequently reduced to the amino compound V

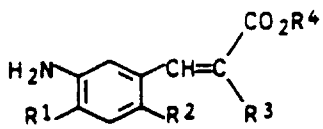

V

and V is condensed with a tetrahydrophthalic anhydride VI

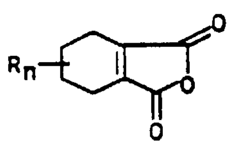

VI.

**3.** A process for the preparation of a compound I as claimed in claim 1, where $R^3$ is chlorine or bromine, wherein a nitrocinnamic ester of the formula IVb

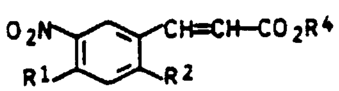

IVb

is halogenated in an inert organic solvent with chlorine or bromine to give the nitrocinnamic ester of the formula IV

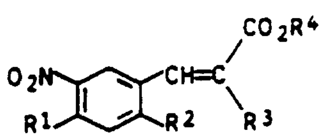

IV,

subsequently reduced to V and condensed with the tetrahydrophthalic anhydride VI according to claim 2.

4. A process for the preparation of a compound I as claimed in claim 1, wherein a cinnamyl chloride of the formula VII

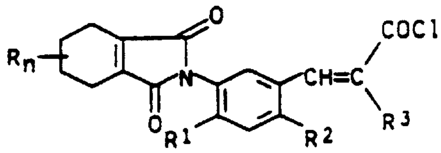

VII

is reacted in a conventional manner in an inert organic solvent with an alcohol of the formula III according to claim 2 to give the cinnamic ester I.

5. A process for the preparation of a compound I as claimed in claim 1, wherein an acetal of the formula VIII

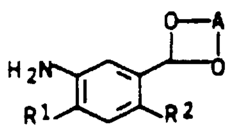

VIII,

where A is an ethylene or propylene unit which can be substituted by one to three $C_1$-$C_3$-alkyl groups, is condensed, with diol elimination, with a tetrahydrophthalic anhydride of the formula VI according to claim 2 to give the aldehyde IX

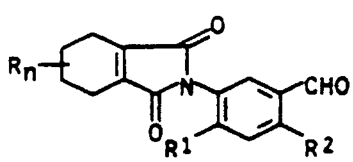

IX

and IX is subsequently reacted with an ylide of the formula X

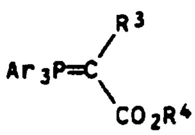

X,

where Ar is aryl.

6. The use of a 5-(N-3,4,5,6-tetrahydrophthalimido)cinnamic ester of the formula I as claimed in claim 1 as a herbicide.

7. A herbicidal agent containing a 5-(N-3,4,5,6-tetrahydrophthalimido)cinnamic ester of the formula I as claimed in claim 1 and inert additives.

8. A herbicidal agent as claimed in claim 7, containing a 5-(N-3,4,5,6-tetrahydrophthalimido)cinnamic ester of the formula I and further active constituents.

9. A method for controlling unwanted plant growth, wherein the unwanted plants and/or their habitat are treated with a herbicidally effective amount of a 5-(N-3,4,5,6-tetrahydrophthalimido)cinnamic ester I as claimed in claim 1.

**10.** A method for the defoliation of cotton, wherein the cotton plants are treated with an effective amount of a 5-(N-3,4,5,6-tetrahydrophthalimido)cinnamic ester I as claimed in claim 1.

**Claims for the following Contracting State : ES**

**1.** A herbicide containing inert additives and a 5-(N-3,4,5,6-tetrahydrophthalimido)cinnamic ester of the formula I

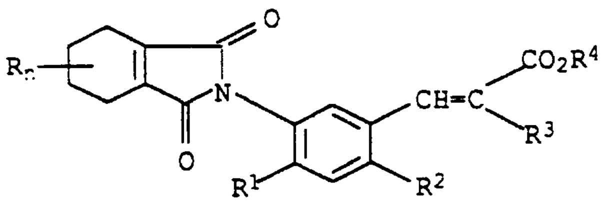

I

where $R^1$ is hydrogen or fluorine, $R^2$ is halogen, $R^3$ is hydrogen, halogen or $C_1$-$C_4$-alkyl, $R^4$ is hydrogen, $C_1$-$C_6$-alkyl which can be substituted by one or two $C_1$-$C_4$-alkoxy or $C_1$-$C_4$-alkylthio groups, or $C_3$-$C_7$-cycloalkyl, $C_3$-$C_6$-alkenyl, $C_3$-$C_6$-alkynyl or benzyl, R is $C_1$-$C_4$-alkyl, and n is 1 or 2.

**2.** A herbicidal agent as claimed in claim 1, containing a 5-(N-3,4,5,6-tetrahydrophthalimido)cinnamic ester of the formula I and further active constituents.

**3.** A process for the preparation of a compound I as claimed in claim 1, wherein a nitrocinnamyl chloride of the general formula II

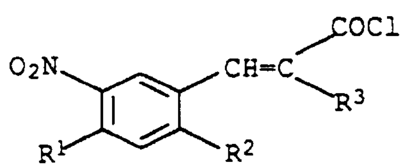

II

is reacted in a conventional manner in an inert organic solvent in the presence of a base with an alcohol of the formula III

$R^4OH$ III

to give the nitrocinnamic ester IV

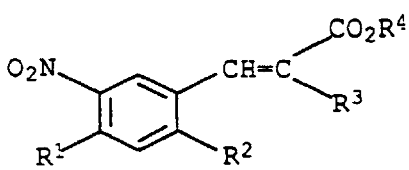

IV,

the nitrocinnamic ester IV is subsequently reduced to the amino compound V

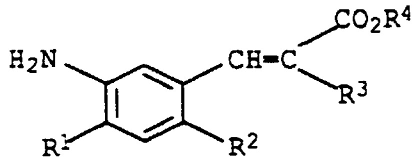

V

and V is condensed with a tetrahydrophthalic anhydride VI

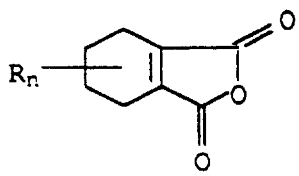

VI.

4. A process for the preparation of a compound I as claimed in claim 1, where $R^3$ is chlorine or bromine, wherein a nitrocinnamic ester of the formula IVb

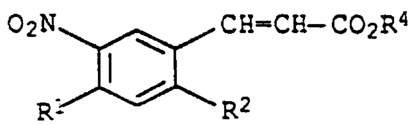

IVb

is halogenated in an inert organic solvent with chlorine or bromine to give the nitrocinnamic ester of the formula IV

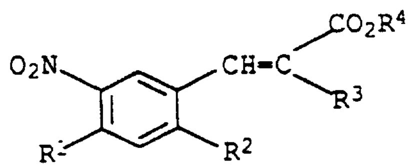

IV,

subsequently reduced to V and condensed with the tetrahydrophthalic anhydride VI according to claim 3.

5. A process for the preparation of a compound I as claimed in claim 1, wherein a cinnamyl chloride of the formula VII

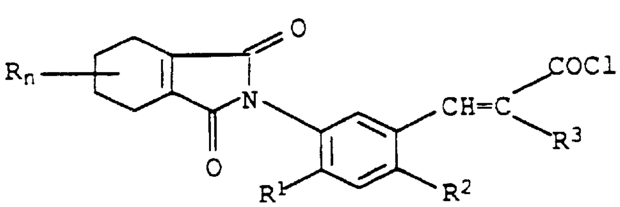

VII

is reacted in a conventional manner in an inert organic solvent with an alcohol of the formula III according to claim 3 to give the cinnamic ester I.

6. A process for the preparation of a compound I as claimed in claim 1, wherein an acetal of the formula VIII

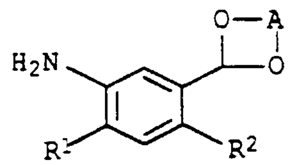

VIII,

where A is an ethylene or propylene unit which can be substituted by one to three $C_1$-$C_3$-alkyl groups, is condensed, with diol elimination, with a tetrahydrophthalic anhydride of the formula VI according to claim 3 to give the aldehyde IX

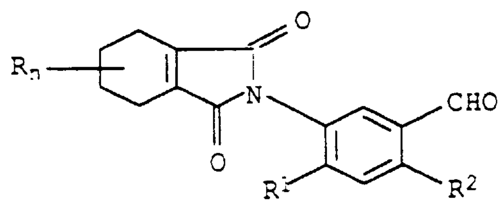

IX

and IX is subsequently reacted with an ylide of the formula X

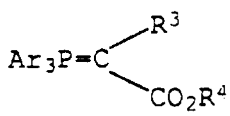

X,

where Ar is aryl.

**7.** The use of a 5-(N-3,4,5,6-tetrahydrophthalimido)cinnamic ester of the formula I as claimed in claim 1 as a herbicide.

**8.** A method for controlling unwanted plant growth, wherein the unwanted plants and/or their habitat are treated with a herbicidally effective amount of a 5-(N-3,4,5,6-tetrahydrophthalimido)cinnamic ester I as claimed in claim 1.

**9.** A method for the defoliation of cotton, wherein the cotton plants are treated with an effective amount of a 5-(N-3,4,5,6-tetrahydrophthalimido)cinnamic ester I as claimed in claim 1.

**Revendications**

**Revendications pour les Etats contractants suivants : AT, BE, CH, DE, DK, FR, GB, IT, LI, LU, NL, SE**

**1.** Ester d'acide 5-(N-3,4,5,6-tétrahydrophtalimido cinnamique de la formule générale

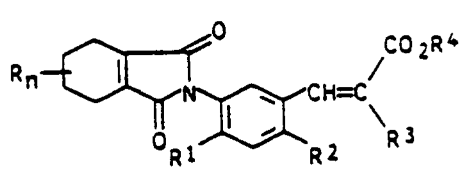

I

dans laquelle les substituants et les indices ont la signification suivante :

- $R^1$ hydrogène et fluor,
- $R^2$ halogène,
- $R^3$ hydrogène, halogène ou un groupe alkyle en C1-C4,
- $R^4$ hydrogène, un groupe alkyle en C1-C6, qui peut être substitué par un ou deux groupes alcoxy en C1-C4 ou (alkyle en C1-C4)-thio, un groupe cycloalkyle en C3-C7, un groupe

alcényle en C3-C6, un groupe alcynyle en C3-C6 ou un reste benzyle,

R alkyle en C1-C4,

n 1 ou 2.

**2.** Procédé de préparation du composé I selon la revendication 1, caractérisé par le fait que l'on transforme un chlorure d'acide nitrocinnamique de la formule générale II

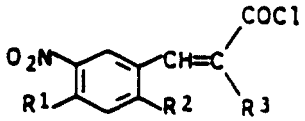

II

de manière connue en soi, dans un solvant organique inerte en présence d'une base avec un alcool de la formule III

$R^4OH$ III

en ester d'acide nitrocinnamique IV

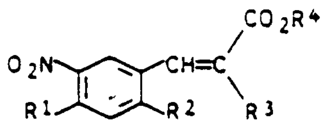

IV

on réduit ensuite l'ester d'acide nitrocinnamique IV en composé amino V

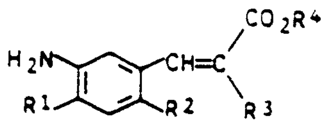

V

et on condense V avec un anhydride d'acide tétrahydrophtalique VI

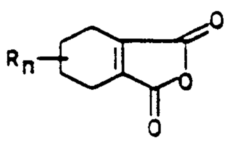

VI

**3.** Procédé de préparation du composé I selon la revendication 1, dans lequel $R^3$ représente chlore ou brome, caractérisé en ce que l'on halogène un ester d'acide nitrocinnamique de la formule IVb

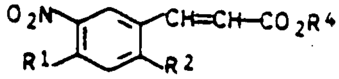

IVb

dans un solvant organique inerte, avec du chlore ou du brome, en ester d'acide nitrocinnamique de formule IV

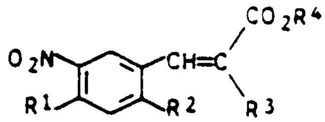

IV

on le réduit ensuite en V et le condense avec l'anhydride d'acide tétrahydrophtalique VI selon la revendication 2.

**4.** Procédé de préparation des composés I selon la revendication 1, caractérisé par le fait que l'on transforme en ester d'acide cinnamique I un chlorure d'acide cinnamique de formule VII

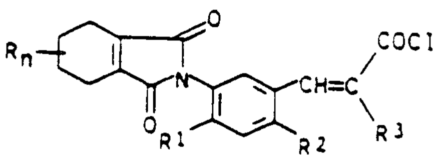

VII

de manière connue en soi, dans un solvant organique inerte, avec un alcool de formule III selon la revendication 2.

**5.** Procédé de préparation des composés I selon la revendication 1, caractérisé par le fait que l'on condense un acétal de formule VIII

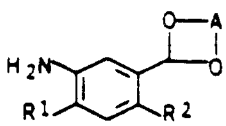

VIII

dans laquelle A représente un motif éthylène ou propylène qui peut être substitué par un à trois groupes alkyle en C1-C3, avec un anhydride d'acide tétrahydrophtalique de formule VI selon la revendication 2, avec élimination du diol, en aldéhyde IX

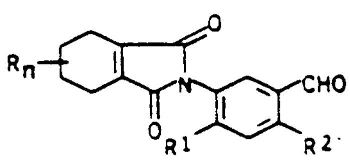

IX

et ensuite on fait réagir IX avec un ylide de formule X

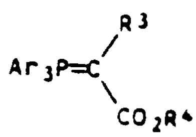

X

dans laquelle Ar représente un reste aryle.

**6.** Utilisation comme herbicide de l'ester d'acide 5-(N-3,4,5,6-tétrahydrophtalimidocinnamique de formule I selon la revendication 1.

**7.** Agent herbicide, contenant un ester d'acide 5-(N-3,4,5,6-tétrahydrophtalimidocinnamique de formule I selon la revendication 1 et des additifs inertes.

8. Agent herbicide selon la revendication 7 contenant un ester d'acide 5-(N-3,4,5,6-tétrahydrophtalimidocinnamique de formule I et d'autres constituants actifs.

9. Procédé de lutte contre une croissance de plantes indésirables, caractérisé par le fait que l'on traite les plantes indésirables et/ou leur biotope avec une quantité à effet herbicide d'un ester d'acide 5-(N-3,4,5,6-tétrahydrophtalimidocinnamique selon la revendication 1.

10. Procédé pour dépouiller le coton de ses feuilles, caractérisé par le fait que l'on traite les plants de coton avec une quantité efficace d'un ester d'acide 5-(N-3,4,5,6-tétrahydrophtalimidocinnamique selon la revendication 1.

**Revendications pour l'Etat contractant suivant : ES**

1. Agent herbicide contenant les additifs inertes et un ester ester d'acide 5-(N-3,4,5,6-tétrahydrophtalimido/cinnamique de la formule générale *I*

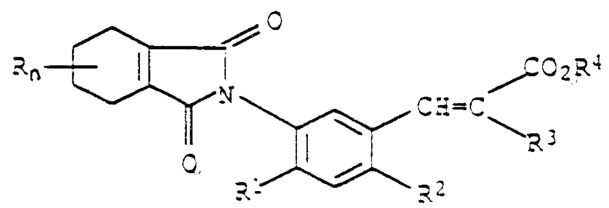

I

dans laquelle les substituants et les indices ont la signification suivante :

- $R^1$ hydrogène et fluor,
- $R^2$ halogène,
- $R^3$ hydrogène, halogène ou un groupe alkyle en C1-C4,
- $R^4$ hydrogène, un groupe alkyle en C1-C6, qui peut être substitué par un ou deux groupes alcoxy en C1-C4 ou alkyle en C1-C4-thio, un groupe cycloalkyle en C3-C7, un groupe alcényle en C3-C6, un groupe alcynyle en C3-C6 ou un reste benzyle,
- R alkyle en C1-C4,
- n 1 ou 2.

2. Agent herbicide selon la revendication 1 contenant un ester d'acide 5-(N-3,4,5,6-tétrahydrophtalimido/cinnamique de formule I et d'autres constituants efficaces.

3. Procédé de préparation du composé I selon la revendication 1, caractérisé par le fait que l'on transforme un chlorure d'acide nitrocinnamique de la formule générale II

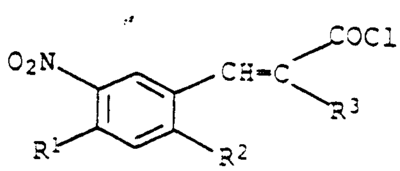

II

de manière connue en soi, dans un solvant organique inerte en présence d'une base avec un alcool de la formule III

$R^4OH$ III

en ester d'acide nitrocinnamique IV

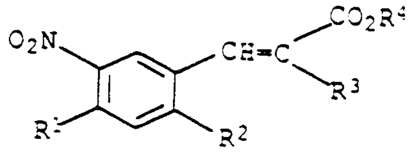

IV

on réduit ensuite l'ester d'acide nitrocinnamique IV en composé amino V

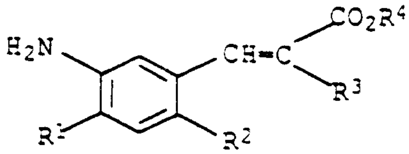

V

et on condense V avec un anhydride d'acide tétrahydrophtalique VI

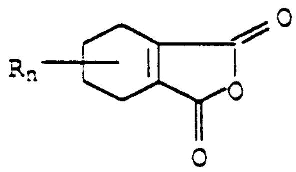

VI

**4.** Procédé de préparation du composé I selon la revendication 1, dans lequel $R^3$ représente chlore ou brome, caractérisé en ce que l'on halogène un ester d'acide nitrocinnamique de la formule IVb

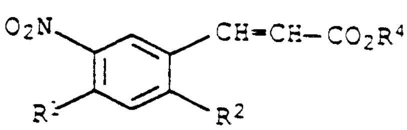

IVb

dans un solvant organique inerte, avec du chlore ou du brome, en ester d'acide nitrocinnamique de formule IV

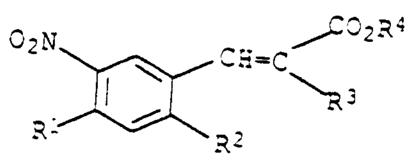

IV

on le réduit ensuite en V et le condense avec l'anhydride d'acide tétrahydrophtalique VI selon la revendication 2.

**5.** Procédé de préparation des composés I selon la revendication 1, caractérisé par le fait que l'on transforme en ester d'acide cinnamique I un chlorure d'acide cinnamique de formule VII

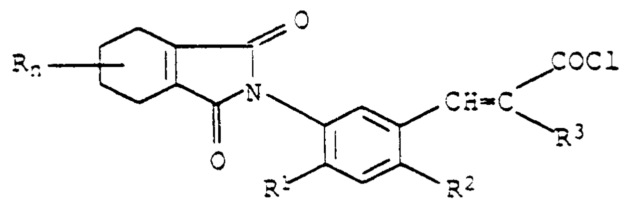

VII

de manière connue en soi, dans un solvant organique inerte, avec un alcool de formule III selon la revendication 2.

**6.** Procédé de préparation des composés I selon la revendication 1, caractérisé par le fait que l'on condense un acétal de formule VIII

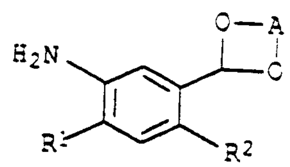

VIII

dans laquelle A représente un motif éthylène ou propylène qui peut être substitué par un à trois groupes alkyle en C1-C3, avec un anhydride d'acide tétrahydrophtalique de formule VI selon la revendication 2, avec élimination du diol, en aldéhyde IX

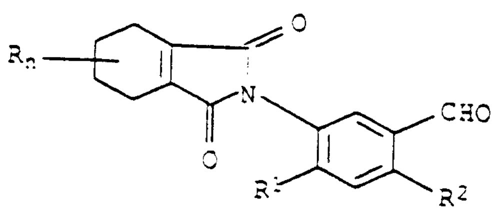

IX

et ensuite on fait réagir IX avec un ylide de formule X

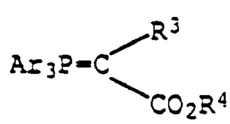

X,

dans laquelle Ar représente un reste aryle.

**7.** Utilisation comme herbicide de l'ester d'acide 5-(N-3,4,5,6-tétrahydrophtalimidocinnamique de formule I selon la revendication 1.

**8.** Procédé de lutte contre une croissance de plantes indésirables, caractérisé par le fait que l'on traite les plantes indésirables et/ou leur biotope avec une quantité à effet herbicide d'un ester d'acide 5-(N-3,4,5,6-tétrahydrophtalimidocinnamique selon la revendication 1.

**9.** Procédé pour dépouiller le coton de ses feuilles, caractérisé par le fait que l'on traite les plants de coton avec une quantité efficace d'un ester d'acide 5-(N-3,4,5,6-tétrahydrophtalimidocinnamique selon la revendication 1.